(19) Europäisches Patentamt
European Patent Office
Office européen des brevets

(11) **EP 2 415 447 A1**

(12) **EUROPEAN PATENT APPLICATION**
published in accordance with Art. 153(4) EPC

(43) Date of publication:
08.02.2012 Bulletin 2012/06

(51) Int Cl.:
*A61K 8/19* (2006.01)  *A61K 8/25* (2006.01)
*A61Q 1/00* (2006.01)  *A61Q 1/02* (2006.01)
*A61Q 1/12* (2006.01)

(21) Application number: 10758670.3

(22) Date of filing: 29.03.2010

(86) International application number:
PCT/JP2010/055611

(87) International publication number:
WO 2010/113899 (07.10.2010 Gazette 2010/40)

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 31.03.2009 JP 2009087883
18.12.2009 JP 2009288452

(71) Applicant: FUJIFILM Corporation
Tokyo 106-8620 (JP)

(72) Inventors:
• NAKAMURA, Kazuhiro
Ashigarakami-gun
Kanagawa 258-8577 (JP)
• KUBO, Toshiaki
Ashigarakami-gun
Kanagawa 258-8577 (JP)

(74) Representative: HOFFMANN EITLE
Patent- und Rechtsanwälte
Arabellastraße 4
81925 München (DE)

(54) **COSMETIC**

(57) The present invention provides a cosmetic composition comprising composite powder grains including: metal oxide flakes having an average thickness in a range of from 50 nm to 200 nm and an aspect ratio in a range of from 10 to 1000; and a coating layer which coat a surface of the metal oxide flakes, and which has a higher refractive index than that of the metal oxide flakes.

EP 2 415 447 A1

**Description**

TECHNICAL FIELD

The invention relates to a cosmetic composition.

BACKGROUND ART

In conventional cosmetic compositions such as makeup cosmetic compositions, pigments having a high covering power, such as titanium oxide or iron oxide, are blended into the cosmetic compositions, or red materials, such as red iron oxide, lake pigments or organic pigments, are added thereto so as to change the skin hue, with a view to covering dullness of the skin color (a state in which the lightness declines, and the chroma of yellow increases) which is perceived with aging due to poor blood circulation or pigmentation,.

However, use of a pigment having a high covering power has a problem of creating an unnatural impression. In order to mitigate the disadvantage, a technique of improving transparency as well as lightness and blueness by blending a blue interference mica into a foundation has been disclosed. (Japanese Patent Application Laid-Open (JP-A) No. 11-139929)
However, the effects exerted by using this technique are insufficient in terms of lightness and chroma, in some cases.

Composite powder grains using metal oxide flakes, and a cosmetic composition using the same are also known which have a high ultraviolet-shielding ability and high transparency with respect to visible light, and which provide a good feeling at use. For example, JP-A No. 7-315859 discloses a metal oxide fine particle-dispersed flake-shaped glass having a thickness of from 0.4 $\mu$m to 0.9 $\mu$m, in which metal oxide fine particles having particle diameters of from 30 nm to 120 nm are dispersed in the form of discrete particles; and a cosmetic composition containing the glass. JP-A No. 9-71417 discloses titanium-oxide-covered metal oxide flake-shaped powder grains in which the surfaces of flake-shaped metal oxide powder grains of from 0.4 $\mu$m to 0.8 $\mu$m having an aspect ratio of from 50 to 94 and being made mainly of amorphous silica are covered with a titanium oxide layer having a thickness of from 25 nm to 95 nm, and also discloses a cosmetic composition containing the powder grains.
However, with the layer structures described in the above documents, the powder grains have a low reflectance. Thus, when the powder grains are blended into a cosmetic composition, the lightness is not sufficient, and there is a problem in that the chroma of the cosmetic composition is deteriorated.

SUMMARY OF INVENTION

Solution to Problem

Accordingly, an object of the present invention is to provide a cosmetic composition of which the lightness and chroma are both favorable.
The invention provides a cosmetic composition.
A first aspect of the invention provides a cosmetic composition comprising composite powder grains, the composite powder grains comprising:

metal oxide flakes having an average thickness in a range of from 50 nm to 200 nm and an aspect ratio in a range of from 10 to 1000; and
a coating layer which coats a surface of the metal oxide flakes, and which has a higher refractive index than that of the metal oxide flakes.

In the cosmetic composition, it is preferred that $\sigma/\mu$, which is the ratio of the standard deviation of the thicknesses of the metal oxide flakes ($\sigma$) to the average value of the thicknesses of the metal oxide flakes ($\mu$) satisfies $\sigma/\mu \leq 0.5$.
The coating layer may include, as a main component, at least one metal oxide selected from titanium oxide, tin oxide, zinc oxide, and zirconium oxide.
The cosmetic composition is preferably a foundation, a makeup base, a face powder, or a point makeup cosmetic composition.

DESCRIPTION OF EMBODIMENTS

The cosmetic composition of the invention is a cosmetic composition including composite powder grains, the composite powder grains including:

metal oxide flakes having an average thickness in a range of from 50 nm to 200 nm and an aspect ratio in a range of from 10 to 1000; and

a coating layer which coats a surface of the metal oxide flakes, and which has a higher refractive index than that of the metal oxide flakes.

Since the cosmetic composition of the invention includes the composite powder grains that include metal oxide flakes, which are coated with a coating layer having a higher refractive index than that of the metal oxide flakes, and have a predetermined shape, the reflectance of each flake particle is very high. Therefore, when the composite powder grains are blended into a skin-color-based cosmetic composition, the lightness of the cosmetic composition as a whole is improved while high chroma of the cosmetic composition is maintained.

In the present specification, the term "process" encompasses an independent process as well as any process which cannot be clearly distinguished from another process, and which attains an expected function of the process of interest.

In the specification, a numerical range expressed using "... to..." denotes a range including numerical values before and after "to" as a minimum value and a maximum value, respectively.

In a case in which plural substances corresponding to a component are present in a composition, the amount of the component in the composition described in the invention refers to the total amount of the plural substances, unless otherwise specified.

The invention is described below.

The metal oxide flakes in the invention are metal oxide flakes having an average thickness in a range of from 50 nm to 200 nm, and an aspect ratio in a range of from 10 to 1000.

By allowing the metal oxide flakes of the composite powder grains to have the shape described above, the reflectance of the metal oxide flakes is increased. Thus, composite powder grains having a high reflection spectrum are formed by providing the metal oxide flakes with the coating layer described below, and favorable lightness and chroma can be obtained.

In the invention, the "metal oxide flakes," means the entire collective entity of the powder grains (individual flakes), and the numerical values of the average thickness, the aspect ratio, and the like mean the average and the aspect ratio of the all metal oxide flakes as a collective entity, respectively.

In the invention, assuming that a plane having the largest area among the planes constituting a single powder grain is a reference plane, the "thickness" of the powder grain means the largest of the lengths of the axes perpendicular to the reference plane, the "transverse length" means the length of the longest major axis on the reference plane, and the "longitudinal length" means the smallest of the lengths of the axes perpendicular to the longest major axis on the reference plane.

The "average particle diameter" as used in the invention refers to a value used generally for tabular particles in the art. Specifically, the average particle diameter is a value obtained by dividing the total of the longitudinal length and the transverse length of a powder grain, which is the total sum of the length in the major axis direction and the length in the minor axis direction, by 2.

The average particle diameter of the metal oxide flakes in the invention is preferably from 50 nm to 150 nm, and more preferably from 70 nm to 120 nm, so as to allow the metal oxide flakes themselves to have increased reflectance over a wide wavelength range of visible light through the principle of thin film interference.

The aspect ratio of the metal oxide flakes is 10 or more in order to exhibit so-called leafing property whereby the metal oxide flakes are arranged along the skin surface when the cosmetic composition of the invention is applied to the skin. When tabular powder grains having high reflectance, such as the composite powder grains in the invention, are used in a cosmetic composition, the particle diameter thereof should be relatively small in order to obtain a natural appearance; otherwise, glittering of the cosmetic composition would be visually observed. Thus, the aspect ratio of the metal oxide flaks is preferably 400 or less, more preferably from 10 to 300. The aspect ratio can generally be measured by observation of the powder grains using scanning electron microscope photography. The aspect ratio of the metal oxide flakes may be, for example, a value obtained by dividing the average particle diameter of 100 powder grains by the average thickness described below.

The average particle diameter of the metal oxide flakes is preferably from 1 $\mu$n to 20 $\mu$m, and more preferably from 10 $\mu$m to 20 $\mu$m. An average particle diameter of the metal oxide flakes of 1 $\mu$m or more provides a sufficient leafing property. An average particle diameter of the metal oxide flakes of 20 $\mu$m or less suppresses glittering.

The metal oxide flakes in the invention preferably have a narrow thickness distribution, and $\sigma/\mu$, which is the ratio of the standard deviation ($\sigma$) of the thicknesses of the metal oxide flakes to the average thickness ($\mu$) of the metal oxide flakes, preferably satisfies $\sigma/\mu \leq 0.5$. When $\sigma/\mu \leq 0.5$ is satisfied, the reflectance or interferential color due to the interference of the metal oxide flakes themselves is reflected in the reflectance or interferential color of the composite powder grains, so that a cosmetic composition having better balanced lightness and chroma can be obtained. In order to balance the lightness and chroma at higher levels, it is more preferable to satisfy $\sigma/\mu \leq 0.3$.

The thickness measurement can be measured by, generally, transmission electron microscope photography after processing the powder grains into ultrathin sections, or scanning electron microscope photography after cutting to form a cross-section. However, the thickness measurement is not limited thereto. The average thickness of the metal oxide flakes can be obtained by taking transmission electron micrographs of the metal oxide flakes, for example, at 10 sites for each of 100 powder grains (individual flakes), measuring the thickness at each of the sites, and averaging the thicknesses at the 1000 sites.

A combination of the average thickness, the aspect ratio, and the average particle diameter of the metal oxide flakes may be any combination of two or more of such parameters. From the viewpoint of obtaining metal oxide flakes having a high reflectance over a wider wavelength range, it is preferable that the metal oxide flakes have an average thickness of from 50 nm to 150 nm and an aspect ratio of from 10 to 400, and optionally further have an average particle diameter of from 1 to 20 $\mu$m. It is more preferable that the metal oxide flakes have an average thickness of from 70 nm to 120 nm and an aspect ratio of from 10 to 300, and optionally further have an average particle diameter of from 1 to 20 $\mu$m.

Examples of the metal oxide flakes in the invention include glass, mica, sericite, talc, silica, titanium dioxide, zinc oxide, and zirconia. These may be used singly, or in combination of two or more thereof. The mental oxide flakes are preferably glass, mica, sericite, talc or silica, or a combination of two or more thereof, from the viewpoint that the metal oxide flakes, when coated with the coating layer described blow, exhibits a favorable reflectance.

The metal oxide flakes in the invention may be produced, for example, from a liquid containing a hydrolyzable/polycondensable organic metal compound, following the description of JP-A Nos. 3-285838 and 4-37622. Specifically, a solution containing a hydrolyzable/polycondensable organic metal compound (precursor solution) is applied to an appropriate substrate, and dried, and then separated from the substrate to form a film, and then sintered, whereby metal oxide flakes can be obtained.

The organic metal compound is preferably a metal alkoxide having an alkoxyl group. Specific examples of the metal in the metal alkoxide include silicon, titanium, aluminum, and zirconium. Examples of the alkoxide in the metal alkoxide include methoxide, ethoxide, propoxide, and butoxide. Such a metal alkoxide is used singly, or in mixture. Since glass flakes are preferably used as the metal oxide flakes in the invention, a silicon alkoxide is preferably used as the organic metal compound. The solvent of the organic-metal-compound-containing solution is not particularly limited as far as the solvent is capable of substantially dissolving the organic metal compound. The solvent is most preferably an alcohol such as methanol, ethanol, propanol, or butanol. The concentration of the organic metal compound in the solvent may be, for example, from 1 to 30% by weight.

The technique for forming the precursor film on a surface of a substrate may be any known technique. For example, methods that may be employed include: a method of immersing a substrate into a liquid containing the organic metal compound and water, and then pulling up the substrate; a method of dropping the liquid onto a substrate, and rotating the substrate at a high speed; and a method of spraying the liquid onto a substrate.

Among the above, from the viewpoint of certainly obtaining metal oxide flakes having a narrow thickness distribution, it is particularly preferable to use a method described in JP-A Nos. 7-315859 and 9-71417, specifically, the pulling-up method.

In the pulling-up method, it is preferable to strictly control factors which affect the thickness, such as the concentration of the precursor, the temperature, the composition of the liquid, and the pulling-up speed, in order to control the thickness precisely.

In a case in which the metal oxide flakes in the invention are natural mica or synthetic mica, the metal oxide flakes can favorably be obtained by exfoliating the mica in layers using a known method. The mica flakes may have a wide thickness distribution, bit are advantageous in terms of costs, compared to glass flakes obtained using a metal alkoxide as a raw material. Moreover, the mica flakes have somewhat decreased surface smoothness as compared with the glass flakes, so that the mica flakes have properties that are less likely to cause glittering. It is also possible to use different types of flake depending on the purposes.

The mica flakes in the invention may be natural mica flakes or synthetic mica flakes. Among them, synthetic mica flakes are particularly preferable for application to the invention due to high transparency thereof. The synthetic mica flakes in the invention can be distinguished from natural mica flakes based on a so-called impurity content in mica. Specifically, synthetic mica flakes can be specified as having a content of Fe, which causes transparency-deteriorating coloration, of less than 0.1% by mass relative to the mass of the mica flakes. The Fe content by mass in mica flakes can be measured generally by using fluorescent X-rays or the like.

The coating layer of the composite powder grains in the invention coats the surface of the metal oxide flakes, and has a higher refractive index than that of the metal oxide flakes.

The compound that forms the coating layer may be any compound that achieves a higher refractive index than that of the metal oxide flakes, with a view to obtaining a high reflectance over a wide wavelength range in the visible wavelength range by the principle of thin film interference. In general, the refractive index in this case is based on a value calculated from a reflectance measurement or an ellipsometer measurement when a thin film is formed on a standard plate of which the refractive index is already known. When the refractive index of the metal oxide flakes as measured in such a manner is, for example, 1.50, the refractive index of the coating layer is higher than 1.50, and is preferably 2.00 or higher, more preferably 2.20 or higher, and even more preferably 2.40 or higher.

The coating layer preferably includes a metal oxide as a main component. Examples of the metal oxide include titanium oxide (anatase-type titanium dioxide having a refractive index: 2.52, rutile-type titanium dioxide having a refractive index of 2.71), zirconium oxide (refractive index: 2.40), tin oxide, zinc oxide (refractive index: 1.95), and a combination of two or more thereof. Among them, the coating layer preferably includes, as a main component, at least one metal oxide selected from titanium oxide, tin oxide and zinc oxide due to their higher refractive indices, and titanium oxide, which has especially high refractive index as compared with the metal oxide flakes, is most preferable. The term "main component" as used herein means a component of which the content is 90% by mass or higher relative to the total mass of the coating layer.

The method of coating the metal oxide flakes with the coating layer may be any method known as wet methods, vapor deposition methods, and the like. For example, a method of hydrolyzing sulfuric-acid-acidic titanium oxysulfate at the boiling temperature (for example, Japanese Examined Patent Application Publication (JP-B) No. 43-25644), and a method of hydrolyzing titanium tetrachloride (for example, JP-B No. 49-3824), are generally known. A stable titanium oxide coating layer having high density can be obtained by, for example, coating a titanium oxide precursor using these methods, and then carrying out thermal treatment at 700 to 1000°C.

The average coating thickness of the coating layer is preferably from 25 nm to 150 nm from the viewpoint of improving the reflectance of the powder grains. When the average thickness is 25 nm or more, an increase in the reflectance of the powder grains can sufficiently be expected. When the average thickness is 150 nm or less, wavelengths giving the local maximum and the local minimum of the reflection spectrum are sufficiently distant from each other, and thus a decrease of the average reflectance of the powder grains over the entire visible light wavelength range can be sufficiently suppressed. The average thickness of the coating layer is more preferably from 40 nm to 90 nm when the compound for forming the coating layer is titanium oxide.

A narrower distribution of the average thickness of the coating layer among the powder grains is more preferable. It is known that the distribution of the average thickness of the coating layer among the powder grains, when expressed by a calculated value obtained by dividing the standard deviation of thicknesses by the average thickness and multiplying the resultant value by 100, can be set to 10% or less, more strictly 5% or less, by using the known method described above, and that an effect in terms of improving the reflectance of the composite powder grains by thin film interference can be exerted.

In a case in which the metal oxide flakes themselves, which serve as bases of the composite powder grains, are macroscopically white:

the composite powder grains obtained are white having high transparency in the thickness direction when the average thickness of the coating layer is less than 40 nm;

the composite powder grains are silver having high transparency when the average thickness of the coating layer is from 40 nm to 60 nm;

the composite powder grains are observed to have a color (interference color) having transparency, in yellow, and then in red, and then in red purple, and then in blue, and then in green, when the average thickness of the coating layer is further increased to 160 nm sequentially; and

the same colors of gold, red, red purple, blue and green are repeatedly observed when the average thickness of the coating layer is further increased.

The metal oxide flake of the invention is colored in a silver to gold color due to an interference effect and the control of the thickness distribution, even in the state of a base alone. Therefore, with an average thickness of the coating layer of from 40 nm to 60 nm, the composite powder grains have a color of from silver having moderate yellow tinge to gold, and lightness can be improved without decreasing chroma; accordingly, an average thickness of the coating layer of from 40 nm to 60 nm is most preferably employed.

The coloration of the metal oxide flakes is clearly different from that of titanium-oxide-coated mica generally widely used in foundations and the like, and provides a stronger transparent feeling, a high reflectance, and a vivid interference color. A reason thereof is that the metal oxide flakes themselves have an interference color based on optical thin film interference with thickness control.

The composite powder grains in the invention include the metal oxide flake and the coating layer described above, and exhibits a strong transparent feeling, high coloring properties, and a high reflectance as described above. Since the average total thickness of the composite powder grains is a value obtained by adding double the thickness of the coating layer to the thickness of the metal oxide flakes, the average total thickness is determined based on an optimal thickness range of the thickness of the metal oxide flake and an optimal thickness range of the coating layer. Accordingly, the average total thickness of the composite powder grains is preferably from 100 nm to 500 nm, more preferably from 120 nm to 300 nm, and even more preferably from 150 nm to 240 nm.

From the viewpoint of exhibiting a high reflectance, and from the viewpoints of lightness and chroma, it is particularly preferable that the composite powder grains in the invention have an average total thickness of from 150 nm to 300 nm and include:

metal oxide flakes having an average thickness of from 70 nm to 120 nm, an aspect ratio of from 10 to 300, and a ratio between an average thickness ($\mu$) and a standard deviation ($\sigma$) of $\sigma/\mu \leq 0.3$; and
a coating layer having an average thickness of from 40 nm to 90 nm.

The cosmetic composition of the invention includes the above-described composite powder grains including the metal oxide flakes having the coating layer. Since the cosmetic composition includes the composite powder grains having a high transparent feeling, high coloring properties, and a high reflectance as described above, the cosmetic composition may be a stable product having high transparency to visible light, both of high lightness and high chroma, and excellent coloring properties.

The amount of the composite powder grains blended into the cosmetic composition of the invention varies depending on the desired type of cosmetic composition, and is in a range of from 1 to 80% by mass, preferably in a range of from 2 to 50% by mass, more preferably in a range of 5 to 30% by mass, with respect to the total mass of solid components including pigments. When the amount of the composite powder grains is 1% by mass or more, lightness, chroma, and coloring properties are sufficiently expected. When the amount of the composite powder grains is 80% by mass or less, sufficient effects can be achieved by addition of other additives, in terms of, for example, color tone change, facilitation of formulation, or an improvement in the adhesion to the skin. In the invention, the term "solid component" means a component which is solid at 25°C and 1 atm.

Besides the composite powder grains, the cosmetic composition of the invention may further include generally used components such as pigment, as necessary- Examples thereof include inorganic pigments such as titanium oxide, zinc oxide, zirconium oxide, yellow iron oxide, black iron oxide, red iron oxide, ultramarine blue, and iron blue; pearl luster pigments such as titanated mica and bismuth oxychloride; powders such as tar dyes, natural colorants, silica beads, and plastic beads of nylon, acrylic, or urethane; extender pigments such as talc, kaolin, mica, and sericite; and other micas, magnesium carbonate, calcium carbonate, aluminum silicate, magnesium silicate, and clays.

It is totally acceptable to subject the composite powder grains to surface treatment so as to modify the powder grains, with a view to improving the dispersibility of the composite powder grains in the cosmetic composition of the invention, or imparting an excellent feel. For example, surface treatment with a so-called hydrophibizing agent changes the surface of glass flakes from hydrophilic to hydrophobic, thereby improving the affinity for an oil added during preparation of the cosmetic composition, or imparting water repellency so as to reduce smudging of make-up. Examples of the hydrophobizing agent include known fluorine-containing surface treatment agents, methylhydrogenpolysiloxane, reactive alkylpolysiloxane, metal soap, and a metal salt of hydrogenated lecithin, acylamino acid, or acylated collagen, the metal being selected from aluminum, magnesium, calcium, titanium, zinc, zirconium, and iron.

If necessary, besides the above-described powder grain components, components that are blended into ordinary cosmetic compositions - such as various oils, surfactants, water-soluble polymers, other powders, moisturizing agents, preservatives, drugs, ultraviolet absorbents, colorants, inorganic salts or organic acid salts, perfumes, chelating agents, pH adjustors, or water - may be blended into the cosmetic composition of the invention as far as the effects of the invention are not impaired.

Examples of oils that can be used include oil components generally used in cosmetic compositions, such as liquid paraffin, Vaseline, paraffin wax, squalane, beeswax, carnauba wax, olive oil, lanolin, higher alcohols, fatty acids, higher fatty acids, ester oils, ceresin, microcrystalline wax, candelilla wax, diglyceride, triglyceride, silicone oil, perfluoropolyether, perfluorodecalin, perfluorooctane, jojoba oil, octyldodecyl myristate, and neopentylglycol dioctanoate.

Examples of surfactants that can be used include surfactants generally used in cosmetic compositions, such as: nonionic surfactants such as polyoxyethylene alkyl ethers, polyoxyethylene fatty acid esters, polyoxyethylene sorbitan fatty acid esters, glycerin fatty acid esters, polyglycerin fatty acid esters, polyoxyethylene glycerin fatty acid esters, polyoxyethylene hydrogenated castor oil, and polyoxyethylene sorbitol farry acid esters; anionic surfactants, typical examples thereof including fatty acid soaps, such as sodium stearate and triethanolamine palmitate; cationic surfactants; and ampholytic surfactants.

Examples of water-soluble polymer that can be used include water-soluble polymers generally used in cosmetic compositions, such as carboxymethylcellulose, methylcellulose, hydroxymethylcellulose, polyvinyl alcohol, polyvinyl pyrrolidone, gum tragacanth, carrageenan, locust bean gum, dextrin, dextrin fatty acid esters, carboxyvinyl polymers, xanthan gum, gelatin, sodium alginate, and gum arabic.

Examples of moisturizing agents that can be used include moisturizing agents generally used in cosmetic compositions, such as sorbitol, xylitol, glycerin, maltitol, propylene glycol, 1,3-butylene glycol, 1,4-butylene glycol, sodium pyrrolidone carboxylate, lactic acid, sodium lactate, and polyethylene glycol.
Examples of preservatives that can be used include preservatives generally used in cosmetic compositions, such as alkyl *p*-oxybenzoates, sodium benzoate, and potassium sorbate.

Examples of drugs that can be used include drugs generally used in cosmetic compositions, such as vitamins, crude drugs, antiphlogistics, and microbicides.

Examples of ultraviolet absorbents that can be used include ultraviolet absorbents generally used in cosmetic compositions, such as *p*-aminobenzoic acid ultraviolet absorbents, anthranyl ultraviolet absorbents, salicylic acid ultraviolet absorbents, cinnamic acid ultraviolet absorbents, and benzophenone ultraviolet absorbents.

Examples of the colorants that can be used include colorants generally used in cosmetic compositions, such as: tar dyes, such as FOOD RED No. 3, FOOD RED No. 104, FOOD RED No. 106, FOOD RED No. 201, FOOD RED No. 202, FOOD RED No. 204, FOOD RED No. 205, FOOD RED No. 220, FOOD RED No. 226, FOOD RED No. 227, FOOD RED No. 228, FOOD RED No. 230, FOOD RED No. 401, and FOOD RED No. 505, FOOD YELLOW No. 4, FOOD YELLOW No. 5, FOOD YELLOW No. 202, FOOD YELLOW No. 203, FOOD YELLOW No. 204, and FOOD YELLOW No. 401, FOOD BLUE No. 1, FOOD BLUE No. 2, FOOD BLUE No. 201, and FOOD BLUE No. 404, FOOD GREEN No. 3, FOOD GREEN No. 201, FOOD GREEN No. 204, and FOOD GREEN No. 205, FOOD ORANGE No. 201, FOOD ORANGE No. 203, FOOD ORANGE No. 204, FOOD ORANGE No. 206, and FOOD ORANGE No. 207; and natural colorants such as carminic acid, laccaic acid, brazilin, and crocin.

Examples of the inorganic salts or organic acid salts include an alkali metal salt, alkali earth metal salt, or aluminum salt of the following: an inorganic acid, such as hydrochloric acid, sulfuric acid, or nitric acid; an oxycarboxylic acid such as citric acid, tartaric acid, lactic acid, or malic acid; a carboxylic acid such as formic acid, acetic acid, or sorbic acid; or an aromatic carboxylic acid such as salicylic acid or benzoic acid.

Specific examples of preferable inorganic salts or organic acid salts include potassium sulfate, sodium sulfate, magnesium sulfate, aluminum sulfate, potassium nitrate, sodium nitrate, magnesium nitrate, aluminum nitrate, calcium nitrate, potassium chloride, magnesium chloride, sodium chloride, calcium chloride, aluminum chloride, potassium carbonate, sodium carbonate, aluminum carbonate, potassium acetate, sodium acetate, calcium acetate, magnesium acetate, sodium formate, potassium formate, magnesium format, sodium citrate, sodium tartrate, potassium sorbate, sodium sorbate, sodium salicylate, potassium benzoate, and sodium benzoate. In particular, potassium sulfate, magnesium sulfate, potassium chloride, magnesium chloride, aluminum chloride, sodium citrate, sodium tartrate, potassium sorbate, sodium salicylate, and sodium benzoate are preferable.

These inorganic salts or organic acid salts may be blended, in a salt state, into the cosmetic composition of the invention. Alternatively, a corresponding acid material and a corresponding base material may be added in stoichiometric amounts necessary for forming a salt, at the time of producing the cosmetic composition of the invention. Water may be added in an arbitrary volume.

Since the cosmetic composition of the invention has both an excellent lightness and an excellent chroma, the cosmetic composition may be used in the form of various types of cosmetic product that includes the composition. The cosmetic composition is preferably used in, for example, a foundation such as a powder foundation, an oily foundation, a creamy foundation, a liquid foundation, or a concealer, a makeup base, or a face powder, and can also be used in a point makeup product such as lipstick or rouge.

Since the cosmetic composition of the invention has excellent lightness as well as excellent chroma, dullness in color can be reduced, and an excellent finish with lightness and transparent feeling can be provided, regardless of application of the cosmetic composition such as a makeup base or a foundation.

EXAMPLES

Hereinafter, the invention will be explained in detail using examples. However, the invention is by no means limited thereto. The word "part(s)" denotes "part(s) by mass" unless otherwise specified.

[Examples 1 to 8, and Comparative Examples 1 to 5]

<Preparation of Composite Powder Grains>

Various types of composite powder grains described in Table 1 were produced according to the following methods.

Among metal oxide flakes, glass flakes were produced by a known method described in, for example, JP-A No. 7-315859 and Japanese Patent No. 3723571.

Specifically, 5400 g of tetramethoxysilane as a glass precursor and 1000 g of 0.1 N hydrochloric acid were dissolved in a 1:1 mixed solvent of ethanol and 2-propanol, so as to have predetermined concentrations. Thereafter, the liquid was agitated at 35°C for 72 hours to prepare a coating liquid. Using pulling-up coating, the coating liquid was coated, with strict thickness control, on a stainless steel support having a smooth surface at a pulling-up rate of 30 to 50 cm/minute, followed by drying at 200°. Thereafter, the coating was separated from the support, and the collected flakes were sintered at 1000°C, and, further, pulverized and classified, as a result of which tabular powder grains (metal oxide flakes) having a desired thickness and a desired aspect ratio described in Table 1 were obtained.

The thickness was adjusted by the tetramethoxysilane concentration and the pulling-up rate through repeated trials and errors.

The thickness of each type of tabular powder grains was measured by taking scanning electron micrographs of 10 sites each for 100 powder grains from a side direction in the state in which the grains were sprayed on a sample stand, measuring the thickness at each of the sites, and then obtaining the average value ($\mu$) of the thicknesses at the 1000 sites by calculation. Further, the standard deviation ($\sigma$) was calculated from the 1000 thickness values and the average thickness ($\mu$), and the thickness distribution was evaluated based on a $\sigma/\mu$ value.

Among metal oxide flakes, synthetic mica was prepared by purchasing a commercially available synthetic phlogopite having a small thickness, followed by further classifying. Specifically, PDM-5L (a synthetic phlogopite having a particle diameter of 7 $\mu$m and an average thickness of 175 nm, and manufactured by Topy Industries, Ltd.) was classified using a known classification means, to obtain tabular powder grains (metal oxide flakes) having a desired thickness and a desired aspect ratio described in Table 1.

The refractive index of the glass fakes was obtained by separately preparing a plate glass having a thickness of 1 mm, and directly measuring the refractive index thereof using an Abbe's refractomer. The refractive index of the coating layer was obtained by separately forming a thin film having a thickness of 100 nm on an optical glass having a known refractive index (BK-7, refractive index = 1.510), measuring a reflection spectrum, and carrying out fitting. With respect to the micas of Examples and Comparative Examples, refractive index values disclosed in literature were used.

The aspect ratio was obtained by spraying each type of tabular powder grains onto a supporting substrate so as to be spread and oriented horizontally, taking a scanning electron micrograph of the sprayed powder grains, measuring the size - specifically, an average particle diameter (an average of the longitudinal length and the transverse length) - of 100 of the powder grains viewed from the above, and then dividing the average value of the 100 powder grains by the average thicknesses calculated by the method described above.

A commercially available mica (manufactured by Merck & Co., Inc.) was used as the mica of Comparative Examples, and the thickness, the aspect ratio, and the like thereof were measured in the same manner as described above.

The coating layer was formed by a known method described in, for examples, JP-A No. 09-71417 and EP 0106235. Specifically, as described in JP-A No. 09-71417, a titanium oxide precursor (a titanium oxysulfate aqueous solution, and sulfuric acid) was added to a dispersion liquid in which the tabular powder grains were dispersed, followed by heating, agitation, filtration, washing, and drying. The precursor was subjected to heating treatment at high temperature (of 1000°C), to form a titanium oxide coating layer.

The average thickness of the coating layer was determined from an average total thickness of the composite powder grains obtained by observation from a side direction using a scanning electron microscope in the same manner as in the case of the average thickness of the metal oxide flakes. The thickness distribution of the coating layer of the composite powder grains was obtained by separately preparing 100 nm-thick super-thin slices, and measuring the coating thicknesses of 10 powder grains, each for 10 viewing fields, using a transmission electron microscope. The standard deviation thereof was divided by the average thickness, and the resultant value was multiplied by 100. The value thus calculated was 10% or less in each case.

Table 1

| | Metal oxide flake | | | | | | Coating layer | | | | Composite powder grains |
| --- | --- | --- | --- | --- | --- | --- | --- | --- | --- | --- | --- |
| | Material | Refractive index | Average thickness $\mu$ (nm) | Standard deviation $\sigma$ (nm) | $\sigma/\mu$ | Aspect ratio | Material | Refractive index | Average thickness (nm) | Interference color | Average total thickness (nm) |
| Example 1 | Glass | 1.5 | 92 | 9.2 | 0.10 | 220 | Titanium oxide | 2.6 | 53 | Gold | 198 |
| Example 2 | Glass | 1.5 | 92 | 9.2 | 0.10 | 220 | Titanium oxide | 2.6 | 70 | Gold | 232 |
| Example 3 | Glass | 1.5 | 92 | 9.2 | 0.10 | 220 | Titanium oxide | 2.2 | 80 | Gold | 252 |
| Example 4 | Glass | 1.5 | 92 | 9.2 | 0.10 | 110 | Titanium oxide | 2.6 | 70 | Gold | 232 |
| Example 5 | Glass | 1.5 | 100 | 10.0 | 0.10 | 110 | Titanium oxide | 2.6 | 70 | Gold | 240 |
| Example 6 | Glass | 1.5 | 80 | 8.0 | 0.10 | 105 | Titanium oxide | 2.6 | 70 | Gold | 220 |
| Example 7 | Mica | 1.58 | 100 | 30.0 | 0.30 | 45 | Titanium oxide | 2.6 | 53 | Gold | 206 |
| Example 8 | Mica | 1.58 | 150 | 45.0 | 0.30 | 33 | Titanium oxide | 2.6 | 53 | Red purple | 256 |
| Comparative Examples 1 | Glass | 1.5 | 230 | 23.0 | 0.10 | 90 | Titanium oxide | 2.6 | 140 | Blue | 510 |
| Comparative Example 2 | Mica | 1.58 | 350 | 175.0 | 0.50 | 150 | Titanium oxide | 2.6 | 57 | Silver | 464 |
| Comparative Example 3 | Mica | 1.58 | 350 | 175.0 | 0.50 | 150 | Titanium oxide | 2.6 | 120 | Red purple | 590 |
| Comparative Example 4 | Mica | 1.58 | 350 | 175.0 | 0.50 | 60 | Titanium oxide | 2.6 | 57 | Silver | 464 |

Preparation of powder foundations>

Components of each of the phase A and the phase B described below were separately weighed out (the numerical values indicating part(s) by mass), and were sufficiently mixed using a Henschel mixer until the colors of the colorants were spread, whereby powder foundations of Examples 1 to 8 and Comparative Examples 1 to 4 were prepared. The type of composite powder grains used in the phase A is shown in Table 1.

| Phase A | | |
|---|---|---|
| | sericite | 47.1 parts |
| | talc | 21.2 parts |
| | titanium oxide | 9.0 parts |
| | iron oxide (yellow) | 1.8 parts |
| | iron oxide (red) | 0.54 parts |
| | iron oxide (black) | 0.27 parts |
| | composite powder grains (Table 1) | 10.0 parts |
| | methyl paraben | 0.09 parts |
| Phase B | | |
| | Dimethicone (&) trimethylsiloxy silicate | 2.985 parts |
| | Dimethylcone (20 cs) | 7.0 parts |
| | Tocopherol | 0.005 parts |
| | Phenoxyethanol | 0.01 parts |

<Comparalive Example 5>

A foundation of Comparative Example 5 (base formulation) was prepared in the same manner as Examples, except that the composite powder grains shown in Table 1 were not added, and were replaced by 6.9 parts of sericite and 3.1 parts of talc.

<Application of Foundation>

Each of the foundations of Examples 1. to 8, and Comparative Examples 1 to 5 was evenly applied, in an application amount of 0.3 mg/cm$^3$, onto a commercially-available skin color speckle sheet (trade name: BIO PLATE, manufactured by Beaulax Co., Ltd., and having a dark skin-colored circular region of 5 grades with respectively different densities over the background having a standard skin color).

<Evaluation of Foundation>

The skin color of a normal region of a sample in which each foundation was applied to the skin color speckle sheet was measured with a colorimeter CR-400 (manufactured by Konica Minolta Sensing, Inc.), and lightness L\*, a\*, b\*, and chroma C\* under a D65 light source were obtained.
Subsequently, the color of the speckle region was measured, and the color difference ΔE\*ab from the normal region was calculated according to the equation described below. The values at the darkest speckle (speckle-1) and the second darkest speckle (speckle-2) among the 5-grade speckles were used to evaluate covering power. The covering power for the sheet was subjected to five-grade evaluated by visual observation (in which Comparative Example 5 was given 3 points while a region on which a foundation was not applied was given 0 point). The results are shown in Table 2.

$$\text{Color difference } (\Delta E*ab) = \{(\Delta L)^2 + (\Delta a)^2 + (\Delta b)^2\}^{1/2}$$

Table 2

| | Colorimeter (D65 light source) | | | | Covering power | | |
|---|---|---|---|---|---|---|---|
| | L* | a* | b* | C* | Speckle-1 | Speckle-2 | Visual observation |
| Example 1 | 73.2 | 7.5 | 18.0 | 19.5 | 6.4 | 6.1 | 4 |
| Example 2 | 72.9 | 6.6 | 23.9 | 24.8 | 5.7 | 5.4 | 5 |
| Example 3 | 72.4 | 6.8 | 23.4 | 24.4 | 6.1 | 5.7 | 5 |
| Example 4 | 72.9 | 6.6 | 23.9 | 24.8 | 5.7 | 5.4 | 5 |
| Example 5 | 72.7 | 6.7 | 23.7 | 24.6 | 5.8 | 5.5 | 5 |
| Example 6 | 73.0 | 6.5 | 24.0 | 24.9 | 5.7 | 5.4 | 5 |
| Example 7 | 73.1 | 7.0 | 24.0 | 25.0 | 6.0 | 5.6 | 5 |
| Example 8 | 72.2 | 8.0 | 17.5 | 19.2 | 6.6 | 5.8 | 4 |
| Comparative Example 1 | 72.0 | 6.0 | 17.0 | 18.0 | 6.7 | 6.2 | 4 |
| Comparative Example 2 | 71.8 | 7.6 | 19.0 | 20.5 | 6.4 | 5.9 | 4 |
| Comparative Example 3 | 70.8 | 8.3 | 19.9 | 21.6 | 8.5 | 7.7 | 2 |
| Comparative Example 4 | 72.0 | 7.4 | 18.0 | 19.5 | 6.3 | 5.9 | 4 |
| Comparative Example 5 | 70.9 | 8.2 | 20.5 | 22.1 | 6.9 | 6.3 | 3 (standard) |
| Not Applied | 70.1 | 8.6 | 21.8 | 23.4 | 14.0 | 13.4 | 0 |

As shown in Table 2, it is demonstrated that each Examples 1 to 8 exhibits excellent lightness and excellent chroma, as well as high covering power.

By contrast, in Comparative Examples 1 to 4 in which the thickness of the metal oxide flakes was large, it is clear that it is not possible to achieve both of excellent lightness and excellent chroma at high levels; in particular, it is demonstrated that even when glass flakes similar to the glass flakes used in Examples were used, the chroma was low (see Comparative Example 1), and the effects of the invention were not obtained.

[Examples 9 to 16 and Comparative Examples 6 to 11]

Components of each of the phase A, the phase B, and the phase C described below were separately weighed out. The type of composite powder grains used in the phase A is shown in Table 1. The phase B was added into a vessel of a homomixer, and, while agitating, the phase A was gradually added thereto. Thereafter, the vessel was heated to 80°C, and agitation was carried out until all of the components of the phase B were melted. Separately, a vessel to which the phase C was added was also heated to 80°C, and agitation was carried out until all components were dissolved. Subsequently, the temperature of the mixture of the phase B and the phase A and the temperature of the aqueous solution of the phase C were both lowered to 50°C, and the phase C was gradually added to the phase B while agitating using a homomixer, and agitation was carried out until sufficient emulsification was achieved. As a result, liquid foundations of Examples 9 to 16 including the same composite powder grains as those of Examples 1 to 8, respectively, liquid foundations of Comparative Examples 6 to 10 including the same composite powder grains as those of Comparative Examples 1 to 5, respectively, and a liquid foundation of Comparative Example 11 including no composite powder grains, were prepared.
Phase A

| | |
|---|---|
| talc, | 3.9 parts |
| titanium oxide | 9.6 parts |
| iron oxide (yellow) | 0.8 parts |
| iron oxide (red) | 0.2 parts |
| iron oxide (black) | 0.02 parts |
| composite powder grains (table 1) | 5.0 parts |

(continued)

Phase B

| | |
|---|---|
| cyclomethicone | 5.0 parts |
| cyclomethicone (&) Dimethicone polyol | 15.0 parts |
| synthetic wax | 0.10 parts |
| arachidyl behenate | 0.3 parts |
| trihydroxystearin | 0.4 parts |
| phenyltrimethicone | 5.0 parts |
| tridecyl isononanoate | 5.0 parts |
| Laureth-7 | 0.5 parts |

Phase C

| | |
|---|---|
| deionized water | 43.0 parts |
| BG | 8.0 parts |
| NaCl | 2.0 parts |
| anhydrous sodium acetate | 0.3 parts |
| phenoxyethanol | 0.2 parts |
| EDTA-2Na | 0.1 parts |

The liquid foundations of Examples 9 to 16 and Comparative Examples 6 to 11 were evaluated in the same manner as the evaluation of the powder foundations described above.
As a result, each of the foundations of Examples 9 to 16 exhibited high lightness and high chroma, thus being favorable; in particular, the foundations of Examples 10 to 15 5 exhibited especially high lightness and especially high chroma, and provided a bright and vivid finish.
By contrast, with the foundations of Comparative Examples 6 to 10, and the ordinarily-formulated liquid foundation (Comparative Example 11) including no composite powder grains, achievement of both of lightness and chroma at such high levels was not made.

[Example 17]

Face powders were prepared in the same manner as Examples 1 to 8, respectively, except that the proportions of the phases B were reduced to 1/3 as compared to the powder foundations of Examples 1 to 8. The face powders obtained were evenly applied, in an application amount of 0.06 g.cm$^{-3}$, onto surfaces to which the liquid foundation base formulation of Comparative Example 11 had been applied, to obtain samples, and the samples were evaluated in the same manner as in Examples 1 to 8.
The face powders obtained exhibited excellent lightness and excellent chroma, similarly to the powder foundations of Examples 1 to 8.

As described above, according to the invention, a favorable cosmetic composition can be obtained, such as a foundation or a face powder, which has excellent lightness and excellent chroma.

The disclosures of Japanese Patent Application No. 2009-087883 filed on March 31, 2009, and Japanese Patent Application No. 2009-288452 filed on December 18, 2009 are incorporated herein by reference in their entirety. All publications, patent applications, and technical standards mentioned in this specification are herein incorporated by reference to the same extent as if each individual publication, patent application, or technical standard was specifically and individually indicated to be incorporated by reference.

**Claims**

1. A cosmetic composition comprising:

   a composite powder grains comprising:

   metal oxide flakes that have an average thickness in a range of from 50 nm to 200 nm and an aspect ratio in a range of from 10 to 1000; and
   a coating layer that coats a surface of the metal oxide flakes and has a higher refractive index than that of the metal oxide flakes.

2. The cosmetic composition of claim 1, wherein the metal oxide flakes have an average thickness within a range of from 50 nm to 150 nm.

3. The cosmetic composition of claim 1, wherein the metal oxide flakes have an average thickness within a range of from 70 nm to 120 nm.

4. The cosmetic composition of any one of claims I to 3, wherein a standard deviation of thicknesses of the metal oxide flakes ($\sigma$) and the average thickness of the metal oxide flakes ($\mu$) satisfy $\sigma/\mu \leq 0.5$.

5. The cosmetic composition of any one of claims I to 3, wherein a standard deviation of thicknesses of the metal oxide flakes ($\sigma$) and the average thickness of the metal oxide flakes ($\mu$) satisfy $\sigma/\mu \leq 0.3$.

6. The cosmetic composition of any one of claims 1 to 5, wherein an average thickness of the coating layer is in a range of from 25 nm to 150 nm.

7. The cosmetic composition of any one of claims 1 to 6, wherein the composite powder grains have an average total thickness in a range of from 100 nm to 500 nm.

8. The cosmetic composition of claim 1, wherein:

   the composite powder grains comprise:

   the metal oxide flakes having an average thickness of from 70 nm to 120 nm,

   an aspect ratio of from 10 to 300, and a ratio between the average thickness ($\mu$) and a standard deviation ($\sigma$) satisfying $\sigma/\mu \leq 0.3$; and
   the coating layer having an average thickness of from 40 nm to 90 nm, and the composite powder grains have an average thickness of from 150 nm to 300 nm.

9. The cosmetic composition of any one of claims 1 to 8, wherein the coating layer comprises, as a main component, at least one metal oxide selected from the group consisting of titanium oxide, tin oxide, zinc oxide, and zirconium oxide.

10. The cosmetic composition of any one of claims 1 to 9, wherein the metal oxide flakes are glass flakes.

11. The cosmetic composition of any one of claims 1 to 10, wherein the metal oxide flakes are synthetic mica.

12. The cosmetic composition of any one of claims 1 to 11, which is a foundation, a makeup base, a face powder, or a point makeup product.

## INTERNATIONAL SEARCH REPORT

| | International application No. |
|---|---|
| | PCT/JP2010/055611 |

**A. CLASSIFICATION OF SUBJECT MATTER**
*A61K8/19*(2006.01)i, *A61K8/25*(2006.01)i, *A61Q1/00*(2006.01)i, *A61Q1/02*
(2006.01)i, *A61Q1/12*(2006.01)i

According to International Patent Classification (IPC) or to both national classification and IPC

**B. FIELDS SEARCHED**

Minimum documentation searched (classification system followed by classification symbols)
A61K8/19, A61K8/25, A61Q1/00, A61Q1/02, A61Q1/12

Documentation searched other than minimum documentation to the extent that such documents are included in the fields searched
Jitsuyo Shinan Koho          1922-1996   Jitsuyo Shinan Toroku Koho   1996-2010
Kokai Jitsuyo Shinan Koho    1971-2010   Toroku Jitsuyo Shinan Koho   1994-2010

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2007/040206 A1 (Dainichiseika Color & Chemicals Mfg. Co., Ltd.), 12 April 2007 (12.04.2007), claims 1, 4; examples 2 to 6 & US 2009/0258251 A     & EP 1932889 A1 & WO 2007/040206 A1     & KR 10-2008-0041299 A & CA 2624382 A          & CN 101278015 A | 1-9,12<br>10,11 |
| X<br>Y | JP 9-77512 A (MERCK JAPAN LTD.), 25 March 1997 (25.03.1997), claims 1, 2, 6; paragraph [0020]; examples 1 to 9 & US 5702519 A          & EP 763573 A2 & CN 1150165 A | 1-9,12<br>10,11 |

☒ Further documents are listed in the continuation of Box C.        ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 May, 2010 (24.05.10) | 01 June, 2010 (01.06.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

## INTERNATIONAL SEARCH REPORT

| International application No. |
| --- |
| PCT/JP2010/055611 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X<br><br>Y | JP 2008-156439 A   (Merck Patent GmbH),<br>10 July 2008 (10.07.2008),<br>claims 1 to 3, 12; paragraphs [0012], [0040]<br>& US 2008/0153922 A1     & EP 1935946 A2<br>& KR 10-2008-0059061 A   & CN 101294008 A | 1-3,6,7,9,<br>10-12<br>10,11 |
| X<br>Y | JP 2004-231613 A   (Kao Corp.),<br>19 August 2004 (19.08.2004),<br>claim 1; paragraph [0011]<br>(Family: none) | 1-10,12<br>10 |
| X | JP 2002-256174 A   (Asahi Glass Co., Ltd.),<br>11 September 2002 (11.09.2002),<br>claims 1, 4 to 7; examples 1, 2<br>(Family: none) | 1-9,12 |
| X<br>Y | WO 2005/028566 A1   (Nippon Sheet Glass Co.,<br>Ltd.),<br>31 March 2005 (31.03.2005),<br>claims 1, 2, 4 to 6; paragraphs [0001], [0011]<br>& US 2007/0032573 A1     & EP 1666541 A1<br>& KR 10-2006-0090226 A   & CN 1856550 A | 1-10,12<br>10 |
| X<br>Y | JP 2002-138019 A   (Kose Corp.),<br>14 May 2002 (14.05.2002),<br>claims 1, 3; paragraph [0016]<br>(Family: none) | 1-10,12<br>10 |
| X<br>Y | JP 9-71417 A   (Nippon Sheet Glass Co., Ltd.),<br>18 March 1997 (18.03.1997),<br>claims 1 to 3; paragraph [0011]<br>(Family: none) | 1-10,12<br>10 |
| X<br>Y | JP 10-114867 A   (Topy Industries Ltd.),<br>06 May 1998 (06.05.1998),<br>claims 1, 3, 4, 6; paragraph [0001]<br>(Family: none) | 1-9,11,12<br>11 |

Form PCT/ISA/210 (continuation of second sheet) (July 2009)

## EP 2 415 447 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11139929 A
- JP 7315859 A
- JP 9071417 A
- JP 3285838 A
- JP 4037622 A
- JP 4325644 B
- JP 49003824 B
- JP 3723571 B
- EP 0106235 A
- JP 2009087883 A
- JP 2009288452 A

16